# EUROPEAN PATENT APPLICATION

(11) **EP 4 481 044 A1**
(43) Date of publication of application: **25.12.2024**
(21) Application number: 23180698.5
(22) Date of filing: 21.06.2023
(51) Int. Cl.: C12N 15/79, C12N 15/67, C12N 15/113, A61K 31/7105

(54) **CELL SPECIFIC TRANSLATION OF RNA MOLECULES**

(71) Applicant: SRTD Biotech, 52428 Jülich (DE)
(72) Inventor: HOFFMANN, Bernd, 52428 Jülich (DE); RATSFELD, Frederik, 52428 Jülich (DE)
(74) Representative: Kuttenkeuler, David

(57) **Abstract**

The invention provides an improved RNA construct that allows for a target cell specific translation of a protein of interest in eukaryotic cells. The invention applies RNA constructs that are introduced directly into eukaryotic cells, and which are only translated into a protein in the presence of a specific trigger RNA sequence. The invention allows for a controlled expression of proteins or any proteinaceous antigen in target cells. The invention is useful for medical applications such as RNA based vaccination approaches to restrict antigen expression to target tissues.

## Description

### FIELD OF THE INVENTION

The invention provides an improved RNA construct that allows for a target cell specific translation of a protein of interest in eukaryotic cells. The invention applies RNA constructs that are introduced directly into eukaryotic cells, and which are only translated into a protein in the presence of a specific trigger RNA sequence. The invention allows for a controlled expression of proteins or any proteinaceous antigen in target cell. The invention is useful for medical applications such as RNA based vaccination approached to restrict antigen expression to target tissues.

### DESCRIPTION

Various techniques have been used to release and activate molecules of interest in a targeted manner in complex organisms such as humans. Two different approaches are of particular interest here.

Based on carrier systems such as liposomes (D. Papahadjopoulos, M. Moscarello, E. H. Eylar, T. Isac, Biochim. Biophys. Acta. 1975, 401, p. 317), molecules (e.g. cancer therapeutic agents) are coupled to or embedded in these carrier systems. Subsequently, the thus functionalized carrier systems are introduced into the body, where the introduction may include either the entire body or only certain parts of the organism. In this case, the carrier systems are ideally provided such that natural body conditions do not influence the stability between the carrier system and the molecule of interest and therefore the release of the molecule is omitted. External factors can now destabilize the carrier systems and thereby induce the release and thus activity of the incorporated molecules. As external factors for the release, local heating of certain body regions (hyperthermia approach) and light- or pH-induced cleavage of specific bonds between the carrier system and the molecule are used. All of these systems are summarized in principle by Qiu and Park (Advanced Drug Delivery Reviews 53 (2001) pp. 321-339 Environment-sensitive hydrogels for drug delivery) and Shamay and colleagues (Biomaterials, Light induced drug delivery into cancer cells 2011 32: pp. 1377-86). However, it is particularly problematic in all of these methods that the functional molecules must be introduced into the entire body at a high concentration and this can often lead to side effects and non-specific releases. In addition, the methodologies operate in a site-specific, but not cell type-specific, manner and thus for example cytostatic agents, in addition to the intended cancer cells, also damage surrounding tissue or are also rapidly distributed from the site of release in the body. Here, too, high doses must be used.

As a second methodology for the targeted release of molecules, systems are usually used in which the molecules of interest are coupled either directly or again by means of carrier systems to ligands which have a high specificity to certain surface molecules of defined cell types. Highly expressed surface receptors of cancer cells such as GPRCs or folate receptors are thus bound by the ligands (Lappano R, Maggiolini M., Nat Rev Drug Discov. 2011, 10: pp. 47-60, G proteincoupled receptors: novel targets for drug discovery in cancer; Sudimack J, Lee R J., Adv Drug Deliv Rev. 2000, 41: pp. 147-62. Targeted drug delivery via the folate receptor). After the ligands are bound to the surface receptors, bound molecules can either directly exhibit their activity (for example, radiopharmaceuticals or MRI contrasting reagents) or can exhibit their function (e.g. chemotherapeutic agents or DNA vectors) after phagocytic uptake into the cells. However, this method also has significant disadvantages, which relate to the again relatively high total concentration in the entire body system, but also have to do specifically with the fact that most surface receptors have no absolute specificity for individual cell types or disease patterns and therefore coupling to such receptors may be associated with increased side effects in healthy tissues. In addition, a variety of specific receptors do not induce phagocytic uptake of the bound carrier systems after coupling, and therefore these carrier systems remain outside the cells.

Direct cell dependent activation of RNA expression has also been shown by ADAR dependent gene editing (Qian et al. Nature, 2022, 610: pp. 713-721; Kaseniit et al. Nature Biotechnology, 2023, 41: pp. 482-487, as completely independent mechanism as shown here as well as by toehold RNAs (Zhao et al. Nature Biotechnology, 2022, 40: pp. 539-545. For the latter, major disadvantages deal with the fact that highly specific sequence motifs have to be designed to interfere with a translation initiator as well as a target cell specific RNA in parallel in a hierarchic kinetic fashion.

WO 2018/149740 describes a system of selectively expressed RNA molecules (seRNA) for efficient targeting and simultaneous functionalization of any cell type. Based on nucleic acids that can be transferred as DNA or RNA in a non-targeted manner, resulting RNA molecules encode for an effector protein but remain inactive in healthy cells while being activated and translated in predefined target cells only. Targeting of diseased cells is therefore transferred from the outside of the cell to the immense intracellular pool of disease specific molecules. This targeting technology is made possible by a unique combination of various highly conserved RNAbased regulatory mechanisms. The optimal arrangement of all individual domains to each other in a simple modular system guarantees for translational inactivity in non-target cells. In contrast, target cells induce partial degradation of the same seRNA molecules by sense-antisense interactions, whereby Internal Ribosomal Entry site (IRES)-blocking sequences are removed to enable IRES-dependent translation while simultaneously inhibiting further degradation. The seRNA constructs were in practise always expressed via a template nucleic acid such as a DNA vector that is introduced into the target cells. The active seRNA is then produced via the transfected cells transcription machinery.

The construct for seRNA expression disclosed in the prior art still depended on the template-based expression (transcription) within the target cell. Furthermore, the seRNA constructs were of a particular length which has a negative effect on efficiency and handling. For example, prior art seRNA constructs required inhibition of CAP dependent translation. Therefore, it is an object of the present invention to improve seRNA construct structure and the methods of their introduction into target cells.

### BRIEF DESCRIPTION OF THE INVENTION

The invention is described by the following brief itemized aspects and embodiments of the invention:
Item 1: A method for a targeted expression of a polypeptide of interest in a eukaryotic target cell, the method comprising the steps of:
   (i) Providing an RNA construct, wherein the RNA construct has at least the following sequence elements (a), (b), (c) and (d) in 5' to 3' direction, wherein
      (a) is an antisense element comprising a sequence that is complementary to at least a portion of a trigger RNA (endogenously) expressed by the eukaryotic target cell;
      (b) is a blocking element that interacts with at least a portion of the translation initiator element (c) at the 3' position;
      (c) is a translation initiator element;
      (d) is a coding element coding for the polypeptide of interest,
   (ii) Directly introducing the RNA construct into a plurality of eukaryotic cells, wherein the plurality of eukaryotic cells comprises at least one eukaryotic target cell and at least one eukaryotic non-target cell;
   wherein the blocking element (b) inhibits the function of the translation initiator element (c) in absence of the trigger mRNA in a eukaryotic cell, and is sterically displaced and thereby does not inhibit the function of the translation initiator element (c) in presence of the trigger mRNA in a eukaryotic cell.
Item 2: The method of item 1, wherein the plurality of eukaryotic cells is characterized in that the eukaryotic target cell compared to the eukaryotic non-target cell expresses higher levels of the trigger RNA; preferably wherein a higher levels is an at least 2 fold higher expression of the trigger RNA, preferably at least an expression that is selected from a 3 fold, 4 fold, 5 fold, 6 fold, 7 fold, 8 fold or higher expression.
Item 3: The method of item 1 or 2 and wherein the eukaryotic non-target cell is characterized by a non-expression of the trigger RNA in the error margins of a biological system.
Item 4: The method of any one of items 1 to 3, wherein the eukaryotic target cell and eukaryotic non-target cell are two independent cellular entities (preferably two different cell types) or wherein the eukaryotic target cell and eukaryotic non-target cell are the same cellular entity or cell type but at different time points and/or under different conditions.
Item 5: The method of any one of items 1 to 4, wherein the construct does not comprise a nuclease resistant or inhibitory element, preferably wherein the construct does not comprise a hairpin between elements (b) and (c).
Item 6: The method of any one of items 1 to 5, which does not comprise a 5' end modification, preferably does not comprise 5' cap.
Item 7: The method of any one of items 1 to 6, wherein the RNA construct comprises in its sequence at least one modified nucleotide.
Item 8: The method of any one of items 1 to 7, wherein the RNA construct is *in vitro* transcribed from a DNA template, and optionally isolated, before step (i).
Item 9: The method of any one of items 1 to 8, wherein translation initiator element is a synthetic or wild type IRES sequence.
Item 10: The method of item 9, wherein the synthetic or wild-type IRES sequence is selected from the group consisting of encephalomyocarditis virus (ECMV) IRES, Coxackievirus B3 (CVB3) IRES, Taura Syndrome Virus (TSV) IRES, Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg marburgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus), eukaryotic IRES derived from Homo sapiens, Aplysia californica, Canis lupus familiaris, Drosophila melanogaster, Gallus gallus, Mus pahari, Mus musculus, Saccharomyces cerevisiae, Zea mays, Rattus norvegicus, Ovis aries, SYTE-IRES1, SYTE-IRES2, and combinations thereof.
Item 11: The method of any one of items 1 to 10, wherein the antisense element comprises, or consists of, a sequence that is at least 90% complementarity to the at least a portion of the trigger RNA.
Item 12: The method of any one of items 1 to 11, wherein the RNA construct does not comprise an upstream open reading frame (uORF).
Item 13: The method of any one of items 1 to 12, wherein the antisense element is capable of specifically binding to the trigger RNA.
Item 14: The method of item 13, wherein the antisense element does not bind to a poly A tail of the trigger RNA.
Item 15: A nucleic acid template vector suitable for in-vitro expressing an RNA construct recited in any one of items 1 to 14.
Item 16: The nucleic acid template vector of item 15, which is a DNA or RNA vector.
Item 17: The nucleic acid template vector, which comprises a (RNA) promoter sequence under operable linkage to an expression cassette encoding the RNA construct.
Item 18: A vaccine composition, comprising the RNA construct recited in any one of items 1 to 14, together with a suitable carrier and/or excipient.
Item 19: The vaccine composition of item 18, wherein the protein of interest is an antigen.
Item 20: An RNA construct for use in the vaccination against a disease in a subject, wherein the RNA construct is as recited in any one of items 1 to 14, and wherein the protein of in interest is an antigen suitable for vaccination against the disease.
Item 21: The RNA construct for use of item 20, wherein the disease is cancer or an infectious disease.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect**, the object of the invention is solved by a method for a targeted expression of a polypeptide of interest in a eukaryotic target cell, the method comprising the steps of:
(i) Providing an RNA construct, wherein the RNA construct has at least the following sequence elements (a), (b), (c) and (d) in 5' to 3' direction, wherein
   (a) is an antisense element comprising a sequence that is complementary to at least a portion of a trigger RNA (endogenously) expressed by the eukaryotic target cell;
   (b) is a blocking element that interacts with at least a portion of the translation initiator element (c) at the 3' position;
   (c) is a translation initiator element;
   (d) is a coding element coding for the polypeptide of interest,
(ii) Directly introducing the RNA construct into a plurality of eukaryotic cells, wherein the plurality of eukaryotic cells comprises at least one eukaryotic target cell and at least one eukaryotic non-target cell;
wherein the blocking element (b) inhibits the function of the translation initiator element (c) in absence of the trigger mRNA in a eukaryotic cell, and is sterically displaced and thereby does not inhibit the function of the translation initiator element (c) in presence of the trigger mRNA in a eukaryotic cell.

The invention shall as an alternative first aspect pertain to a method of designing and/or producing/providing an RNA construct as selective expression RNA (seRNA), which is suitable for a targeted expression of a polypeptide of interest in a eukaryotic target cell, the method comprising the steps of:
(i) Designing or producing an RNA construct, wherein the RNA construct has at least the following sequence elements (a), (b), (c) and (d) in 5' to 3' direction, wherein
   (a) is an antisense element comprising a sequence that is complementary to at least a portion of a trigger RNA (endogenously) expressed by the eukaryotic target cell;
   (b) is a blocking element that interacts with at least a portion of the translation initiator element (c) at the 3' position;
   (c) is a translation initiator element;
   (d) is a coding element coding for the polypeptide of interest,
(ii) formulating the RNA construct for a direct introduction of the RNA construct into a plurality of eukaryotic cells, wherein the plurality of eukaryotic cells comprises at least one eukaryotic target cell and at least one eukaryotic non-target cell;
wherein the blocking element (b) inhibits the function of the translation initiator element (c) in absence of the trigger mRNA in a eukaryotic cell, and wherein the interaction of the blocking element with the translation initiator element is inhibited, for example the blocking element is sterically displaced, and thereby does not inhibit the function of the translation initiator element (c) in presence of the trigger mRNA in a eukaryotic cell.

Upon introduction of the RNA construct of the invention into a target cell, the trigger RNA that is present in such target cell, will via base pairing hybridize with the antisense element and thereby initiate a displacement of the blocking element and thereby activates the IRES of the RNA construct. The result is a ribosomal translation of the coding element into the protein of interest.

The term trigger RNA in context of the present invention shall be understood as an RNA molecule that is endogenously expressed in a eukaryotic target cell. A trigger RNA can be any type of expressed RNA that is either specific for the target cell or is specifically higher expressed in the target cell. For example, the invention provides a method for the targeted expression which is characterized in that the eukaryotic target cell compared to the eukaryotic non-target cell expresses higher levels of the trigger RNA; preferably wherein a higher levels is an at least 2 fold higher expression of the trigger RNA. More preferably is at least an expression that is selected from a 3 fold, 4 fold, 5 fold, 6 fold, 7 fold, 8 fold, 10 fold, 100 fold, 1000 fold or higher expression in the eukaryotic target cell compared to the eukaryotic non-target cell. In some preferred embodiments of the invention the eukaryotic non-target cell is characterized by a non-expression of the trigger RNA in the error margins of a biological system.

A trigger RNA may be selected from any RNA molecule, but should allow for a differentiation of a target from a non-target cell. Types of RNA are Messenger RNA (mRNA), which carries genetic information for protein synthesis, transfer RNA (tRNA) delivers specific amino acids to the ribosomes, ribosomal RNA (rRNA) which is a structural component of ribosomes, small nuclear RNA (snRNA) which aids in mRNA splicing, small nucleolar RNA (snoRNA) which guides RNA modifications, microRNA (miRNA) which regulates gene expression, long non-coding RNA (IncRNA) (also influences gene expression and cellular signalling), and circular RNA (circRNA) which participates in gene regulation.

The eukaryotic target cell and eukaryotic non-target cell may be two independent cellular entities (preferably two different cell types, or differently differentiated cells) or wherein the eukaryotic target cell and eukaryotic non-target cell are of the same cellular type, or differentiation status, but at different time points and/or under different conditions. For example, a target cell may be a cell of a certain type that is in contact with a ligand molecule that influences RNA expression, while the non-target cell is a similar cell, but which is not under the influence of the same ligand. The person of skill may envision depending on the application of the method further situation which allows a selective expression of the protein of interest based in differentially expressed trigger RNA.

In certain preferred embodiments of the invention, the construct does not comprise a nuclease resistant or inhibitory element, preferably wherein the construct does not comprise an inhibitors element, such as a hairpin, between elements (b) and (c).

Prior art seRNA construct use such inhibitory elements in order to restrict the complete degradation of the introduced mRNA to specific areas thereof after binding of sense and antisense/RNAi mRNA motifs. Prior art constructs were designed usually with the intention to induce RNA degradation by RNA interference to remove the blocking element. However, in order to avoid a full degradation of the seRNA mRNA sequence motifs must be incorporated which interrupt the degradation due to binding partners or secondary structures. Such sequence motifs may include hairpin loops or stem loops. They can be used in single or multiple alignment in both the 5' and the 3' direction for sense-antisense pairing. The use of sequence motifs, which are typically stabilized by secondary structures or binding partners, thus leads, in combination with the specific antisense/RNAi cassette in target cells with sense pairing, to the interruption of mRNA degradation and thereby to stabilization of the RNA motifs located in the 3' direction towards this sequence. The stable secondary structures then assume the function of the CAP with respect to stabilization, but not with respect to translation initiation. In preferred embodiments, the RNA constructs of the present invention are devoid of such inhibitory structures between elements (b) and (c).

Similarly, the use of RNA constructs in accordance with the invention do not require any typical 5' or 3' modification of the RNA in contrast with prior art molecules. Therefore, in certain embodiments, the RNA construct of the invention do not comprise a 5' end modification and/or do not comprise a 3' end modification. Typical 5' end modification includes 5' cap structures. A typical Cap sequence may be a natural 7-methylguanosine (m7G) cap or may also contain chemical or other modifications. For certain applications, the invention avoids the use of cap sequences. Preferably the RNA constructs of the invention do not contain any translation initiating 5' elements, and shall not contain 3' poly A sequences. However, such embodiments may specifically include the use of stabilizing modifications of either or both the 3' and 5' end of the RNA construct. Such modifications are well known to the skilled artisan.

RNA molecule terminal structures such as Caps that may be used in the RNA constructs of the invention in certain embodiments are such structures that are used for the stabilization of a (linear) RNA construct. These are for example: commercially available Caps for 5` stabilization: are GP₃G, m⁷GP₃G, m₂^{7,3'-O}GP₃G (ARCA), and Clean Cap, Published Caps for 5` stabilization are: b⁷GP₃G, e⁷GP₃G, m₂^{2,7}GP₃G, m₃^{2,2,7}GP₃G, m⁷GP₃2'dG, m⁷GP₃m^{2'-O}G, m⁷GP₃m⁷G, m⁷GP₄G, b⁷GP₄G, b⁷m^{3'-O}GP₄G, m₂^{2,7}GP₄G, m₃^{2,2,7}GP₄G, b⁷m²GP₄G, m⁷GP₄m⁷G, m₂^{7,2'-O}GP₃G, m⁷GTP, m₂^{7,3'-*O*}GP₄G, m₂^{7,2'-O}GP₄G , m₂⁷2'dGP₄G, m₂⁷GP₄, m⁷GP₅G, m₂^{7,3'-O}GP₅G, m⁷GP₅, m⁷GP₆G, m2^{7,2'-*O*}GP₆G, m⁷GPPCH₂PG, m⁷GPCH₂PPG, m₂^{7,3'-*O*}GPPCH₂pG, m₂^{7,3'-*O*}GPCH₂PPG, m₂^{7,2'-*O*}GPPCH₂PG, m⁷G P₃CH₂PG, m⁷GPPCH₂PPG, m⁷GPCH₂ P₃G, m₂^{7,2'-*O*}G P₃CH₂PG, m₂^{7,2'-*O*}GPPCH₂PPG, m₂^{7,2'-*O*}GPCH₂ P₃G, m⁷GPPCH₂P, m⁷G P₃CH₂P, m⁷G P₃SG, m⁷GPPSPG, m⁷GPSPPG, m₂^{7,2'-*O*}GP₃SG, m₂^{7,2'-*O*}GPPSPG, m₂^{7,2'-*O*}GPSPPG, m₂^{7,2'-*O*}GP₄SG, m₂^{7,2'-*O*}G P₃SPG, m₂^{7,2'}-OGPPSPPG, m27,2'-OGPSPP3G, m27,2'-OGPPSePG and m7GTPαS.

Such modifications are well known to the skilled artisan. Sequence motifs for 5 and 3'stabilization include: xrRNA, Apt-eIF4G, PolyAC, PABPv1, PABPv2, PABPv3, PABPv4, PCBPv1, PCBPv2, PCBPv3, PCBPv4, YTHDFv1, YTHDFv2, YTHDFv3, YTHDFv4, HBA1 short 3'UTR, HBA1 full 3'UTR, HBA2PR, HBA2MR, 15-lipoxygenase MR, α(I)-collagen MR, tyrosine hydroxylase MR, mouse α-globin 3'UTR, human β-globin 3'UTR, AES motif, mtRNR1 motif, RPS27A3'UTR, Sindbis virus HuR BR, and Hairpin sequences, such as shown in SEQ ID NO 3 or 4.

Such sequence motifs in some embodiments of the invention are included.

The construct of the invention in preferred embodiments also do not require the use of 3' end modifications such as a poly (A) tail, which refers to a 3' homopolymeric tail of adenine nucleotides, which can vary in length (e.g., at least 5 adenine nucleotides) and can be up to several hundred adenine nucleotides). The inclusion of a 3' poly(A) tail can protect the synthetic, modified RNA from degradation in the cell and is a characteristic of the seRNA of the prior art. The present invention is preferred embodiments does not comprise an RNA construct with a 3' end having more than 5 terminal consecutive adenine nucleotides.

To improve for example stability of the RNA construct of the invention one or more types of modified nucleosides may or other chemical modifications of the RNA molecule may be introduced. An RNA construct of the invention therefore may comprise in its sequence at least one modified nucleotide/nucleoside.

As used herein, the terms "synthetic, modified RNA" or "modified RNA" or similar expressions refer to an RNA molecule produced in vitro, which comprise at least one modified nucleoside as that term is defined herein below. The synthetic, modified RNA composition does not encompass mRNAs that are isolated from natural sources such as cells, tissue, organs etc., having those modifications, but rather only synthetic, modified RNAs that are synthesized using in vitro techniques. However, the term shall also encompass isolated RNAs which are subsequently modified. The term "composition," as applied to the terms "synthetic, modified RNA" or "modified RNA," encompasses a plurality of different synthetic, modified RNA molecules (e.g., at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40, at least 50, at least 75, at least 90, at least 100 synthetic, modified RNA molecules or more).

As used herein the term "modified nucleoside" refers to a ribonucleoside that encompasses modification(s) relative to the standard guanine (G), adenine (A), cytidine (C), and uridine (U) nucleosides. Such modifications can include, for example, modifications normally introduced post-transcriptionally to mammalian cell mRNA, and artificial chemical modifications, as known to one of skill in the art.

Modified nucleosides may include 5-methylcytidine (5mC), N6-methyladenosine (m6A), 3,2' -O-dimethyluridine (m4U), 2-thiouridine (s2U), 2' fluorouridine, pseudouridine, 2' -O-methyluridine (Um), 2' deoxy uridine (2' dU), 4-thiouridine (s4U), 5-methyluridine (m5U), 2' - O-methyladenosine (m6A), N6,2' -O-dimethyladenosine (m6Am), N6,N6,2' -O-trimethyladenosine (m62Am), 2' -O-methylcytidine (Cm), 7-methylguanosine (m7G), 2' -O-methylguanosine (Gm), N2,7-dimethylguanosine (m2,7G), N2,N2,7-trimethylguanosine (m2,2,7G), and inosine (I). In one embodiment of this aspect, the at least two modified nucleosides are 5-methylcytidine (5mC) and pseudouridine.

The RNA according to the invention can be produced in a manner known per se. A method wherein the mRNA according to the invention is produced by in vitro transcription from a mixture of ATP, CTP, GTP and UTP.The nucleosides to be used can have modifications such as are also to be found in naturally occurring nucleosides, e.g. methylations or binding variations, but also "synthetic", i.e. not occurring in nature, modifications or a mixture of nucleosides with natural and/or synthetic modifications can be used. Thus, naturally modified nucleosides of at least one type can be combined with synthetically modified nucleosides of the same type or another type or else naturally and synthetically modified nucleosides of one type with only naturally, only synthetically or mixed naturally/synthetically modified nucleosides of another type, where "type" here refers to the type of the nucleosides, i.e. ATP, GTP, CTP or UTP. In many cases, as stated above, for the improvement of immunogenicity and stability or for adjustment of properties it can be beneficial to combine modified nucleosides with functional groups, which provide binding sites, with non-functionally modified nucleosides. The most suitable type or combination can easily be found by those skilled in the art by routine experiments such as are for example also stated below.

In a preferred embodiment the RNA construct is *in vitro* transcribed from a DNA template, and optionally isolated, before step (i).

Translation initiation elements suitable for use in context of the herein disclosed invention are well known in the pertinent art, and can be both a synthetic or wild type IRES sequence. Such synthetic or wild-type IRES sequence may be selected from the group consisting of encephalomyocarditis virus (ECMV) IRES, Coxackievirus B3 (CVB3) IRES, Taura Syndrome Virus (TSV) IRES, Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg marburgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus), eukaryotic IRES derived from Homo sapiens, Aplysia californica, Canis lupus familiaris, Drosophila melanogaster, Gallus gallus, Mus pahari, Mus musculus, Saccharomyces cerevisiae, Zea mays, Rattus norvegicus, Ovis aries, SYTE-IRES1, SYTE-IRES2, and combinations thereof.

An antisense element in accordance with the invention is a sequence that is capable of being bound by a trigger RNA under conditions within the cytoplasm of the eukaryotic target cell. The antisense element in preferred embodiments comprises, or consists of, a sequence that is at least 90% complementarity to at least a portion of the trigger. An antisense element comprises an antisense sequence 40, more preferably at least 50 nucleotides. Preferably such sequences comprise natural antisense sequences (NAT) or correspond to them in their function. An antisense element of the invention is typically capable of specifically binding to the trigger RNA. To identify suitable antisense elements having the best possible antisense function, different parameters can be taken into consideration. For antisense pairing, see, by way of example antisense pairing in the case of RNA interference: Elbashir S M, Harborth J, Lendeckel W, Yalcin A, Weber K, Tuschl T. Duplexes of 21-nucleotide RNAs mediate RNA interference in cultured mammalian cells. Nature. 2001 May 24; 411 (6836): pp. 494-8. Elbashir S M, Lendeckel W, Tuschl T. RNA interference is mediated by 21- and 22-nucleotide RNAs. Genes Dev. 2001 Jan. 15; 15 (2): pp. 188-200. Reynolds A, Leake D, Boese Q, Scaringe S, Marshall WS, Khvorova A. Rational siRNA design for RNA interference. Nat Biotechnol. 2004 March; 22 (3): pp. 326-30. Preferably, the antisense elements of the invention should avoid sequences which are generic to many expressed RNAs, such as a poly tail sequence.

The prior art seRNA constructs required use of 3' and 5' untranslated regions for increasing mRNA stability, and in particular the use of sequence elements that inhibit CAPdependent translation, such as uORFs or similar elements such as sequence motifs forming a quadruplex (e.g. G rich motifs). The constructs of the invention are directly introduced into cells and do not require such regions. For example, in order to increase stability, the invention may use modified nucleosides as described and thereby can reduce construct length significantly.

The length of the coding element used according to the invention depends on the gene product or protein or protein fragment which is to be expressed in the target cells. Hence the coding element can be very short, e.g. have only 20 or 30 nucleotides, or else corresponding to the length of the gene have several thousand nucleotides. Those skilled in the art can select the suitable sequence each time in the usual way.

The RNA constructs are introduced into the plurality of eukaryotic cells via known RNA carrier systems. Those systems may be used therapeutically. The choice of RNA delivery system depends on factors such as the type of RNA, target cell type, desired duration of gene expression, and potential off-target effects. Each method has its advantages and limitations, and ongoing research aims to develop more efficient and specific RNA delivery systems for therapeutic applications. Known approaches include:
- Lipid-Based Delivery Systems: Lipid nanoparticles (LNPs) are widely used for RNA delivery. These lipid-based carriers encapsulate RNA molecules and protect them from degradation in the extracellular environment. LNPs can fuse with the cell membrane, facilitating RNA release into the cytoplasm. They are particularly effective for delivering small interfering RNA (siRNA) and mRNA
- Viral Vectors: Viral vectors, such as lentiviruses and adenoviruses, can be modified to carry therapeutic RNA. These vectors have the ability to efficiently enter target cells and deliver RNA payloads directly into the cell nucleus. Viral vectors can be engineered to reduce their pathogenicity while retaining their delivery capabilities.
- Electroporation: Electroporation involves applying brief electrical pulses to cells, creating temporary pores in the cell membrane. This allows the entry of RNA molecules into the cells. Electroporation is a versatile method suitable for a wide range of cell types, including primary cells and stem cells.
- Cell-Penetrating Peptides (CPPs): CPPs are short peptides that can facilitate the transport of RNA across the cell membrane. They can be conjugated to RNA molecules, forming complexes that can enter cells through various mechanisms, including endocytosis or direct membrane translocation.
- Physical Methods: Physical methods, such as microinjection or biolistic particle delivery (gene gun), involve directly introducing RNA molecules into target cells. These methods are often used for specific applications, such as the delivery of RNA to individual cells or tissues in research settings.
- Exosomes: Exosomes are naturally occurring extracellular vesicles that can be harnessed for RNA delivery. They are secreted by cells and can carry various molecules, including RNA. By engineering exosomes or loading them with therapeutic RNA, they can be utilized as vehicles for targeted delivery to recipient cells.
- Chemical Modification: Chemical modification of RNA molecules can enhance their stability, cellular uptake, and intracellular delivery. These modifications can include conjugating RNA with ligands that specifically bind to cell surface receptors, facilitating receptor-mediated endocytosis.

The seRNA constructs of the invention can be in vitro transcribed or alternatively be chemically synthesized, or combinations thereof such as in vitro transcription with subsequent chemical modification of the transcribed RNA. Methods for chemically polymerization of RNA molecules are well known to the skilled artisan.

In some embodiments, the invention includes the use of self-replicating or circular RNAs in order to facilitate production of bulk RNA constructs in context of the invention. As used herein, the term "self-replicating RNA," or (srRNA) refers to an RNA molecule designed from RNA viruses lacking sequences encoding for structural virus proteins such that it can direct its own replication amplification in a cell, without producing a progeny virus. For example, a srRNA can comprise 5' and 3' alphavirus replication recognition sequences, coding sequences for virus nonstructural proteins, a heterologous gene encoding an antigen and the means for expressing the antigen, and a polyadenylation tract.

The term "circular RNA" has to be understood as an RNA molecule that does not comprise a 5' and a 3' terminus that are typically present in linear RNA molecules (e.g. mRNA). In contrast to mRNA, a circular RNA does not comprise a 5' Cap structure or a 3' terminal tail. In circular RNA, the 3' and 5' ends that are normally present in a linear RNA molecule are joined together. Accordingly, a circular RNA can be considered as an RNA having a closed continuous loop. In the context of the invention, the circular RNA comprises the herein described seRNA structure.

In a **second aspect,** the invention pertains to a nucleic acid template vector suitable for in-vitro expressing an RNA construct disclosed herein.

Typical nucleic acids are DNA or RNA which are suitable for an in vitro expression of the RNA construct of the invention. The nucleic acid template vector of the invention may preferably comprise ab (RNA) promoter sequence under operable linkage to an expression cassette encoding the RNA construct.

In another aspect the invention pertains to pharmaceutical composition comprising the RNA construct recited herein, together with a pharmaceutical acceptable carrier and/or excipient. The pharmaceutical composition of the invention should be formulated such that the RNA construct is delivered to the eukaryotic target cell which is associated with the pathology of a disease.

Preferably the composition of the invention is a vaccine composition. Vaccine compositions of the invention comprise an RNA construct for the targeted expression of an antigen as protein of interest.

In some aspects, the invention further pertains to a method of treating or preventing (vaccination) a disease in a subject, wherein the method comprises the targeted expression of the first aspect. In other embodiments and aspects, the invention pertains to an RNA construct for use in the vaccination against a disease in a subject, wherein the RNA construct is as recited in the preceding aspects. The protein of interest is preferably a protein having a therapeutic effect in cells associated with the disease. For example, the protein of in interest is an antigen suitable for vaccination against the disease.

Typical diseases of the invention include cancer or infectious diseases. Further diseases specifically treatable with the methods of the invention are genetic diseases that require the expression of a protein in certain cell types associated with the disease. For example, genetic diseases that can be treated by introducing wild type or enhanced protein (such as an enzyme) into a subject by gene therapy can be targeted using the methods of the invention.

Hence, the invention pertains to an **RNA construct for use in the treatment or prevention of a disease** in a subject, wherein the RNA construct is as recited herein above, or in any embodiment or item above, and wherein the protein of in interest is an antigen suitable for vaccination against the disease to be prevented or is a protein involved with the pathology of a disease to be treated.

Preferably, the treatment or prevention comprises a method as described herein in the first or second aspect.

Preferred medical applications of the invention include:
For cancer treatment, cancer cell specific RNA molecules can be targeted to activate expression of therapeutically active polypeptides. These peptides typically specifically kill the target cell by inducing apoptosis.

In the treatment of glioblastoma, glioblastoma cells are selectively targeted by their keratin mRNA. Keratin mRNA is not expressed by healthy brain tissue, so here the seRNA of the invention stays inactive. Activation of seRNA leads to expression of constitutive active Caspase3 (effector), which induces cell death specifically in cancer cells. Hence, a protein of interest for targeted expression may be selected from any effector caspases, such as caspase 3 or 8.

Hepatocellular carcinoma cells are selectively targeted by their PGC or LIN28b RNA. These RNAs are not expressed in healthy liver tissue, so here the seRNA stays inactive. Activation of seRNA in cancer cells leads to expression of constitutive active Caspase3 (effector), which induces the cell death of the cancer cells.

The invention can be used to activate a subject's immune system. For example, target cells (e.g. cancer/virus infected) activate seRNA by a specific RNA to induce seRNA dependent expression of an immunogenic protein (e.g. Sars-Cov-2 RBD Domain/ measles/ VZV) (effector). This immunogenic protein is recognized by the cells and degraded. Small sequences of the protein are presented on the cell surface by MHC1 receptors to activate the immune system against the cells. Upon use of common epitopes, e.g. already established vaccinations from childhood can be "re-used" to now target cells of interest.

This technique could be used in combination with a vaccine against virus and/or in combination with classical small molecule drugs.

Treatment of genetic diseases by gene therapy. Cells affected by a genetic disease are targeted by a specific trigger RNA (e.g. Epidermolysis bullosa/ cystic fibrosis). The activation of the seRNA of the invention by the trigger RNA leads to expression of the Cas9 protein (effector). Simultaneously, a gRNA is applied to guide the Cas9 to the sequence of interest to repair the mutation at that site. Since Cas9 is characterized by high background activity, which is inducing unwanted site mutations, expression of Cas9 in target cells only reduced the potential risk to induce secondary cancer events in healthy cells.

Diagnostic approaches. In bladder cancer, urine is centrifuged to isolate containing epithelial bladder cells. These cells are transfected with seRNAs that specifically respond to bladder cancer RNAs. Upon activation an easy to detect reporter protein (e.g. eGFP) (effector) is expressed.

Infectious diseases such as viral diseases can be treated. For example, Hepatitis B virus infections. HBS infected cells are selectively targeted by the HBx RNA sequence. Activation of seRNA leads to expression of constitutive active Caspase3 (effector), which induce the cell death of the infected cells.

Treatment of dysplasia. Affected cells are selectively targeted by HPV16 E6 RNA. Activation of seRNA leads to expression of constitutive active Caspase3 (effector), which induce the cell death of the cancer cells.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

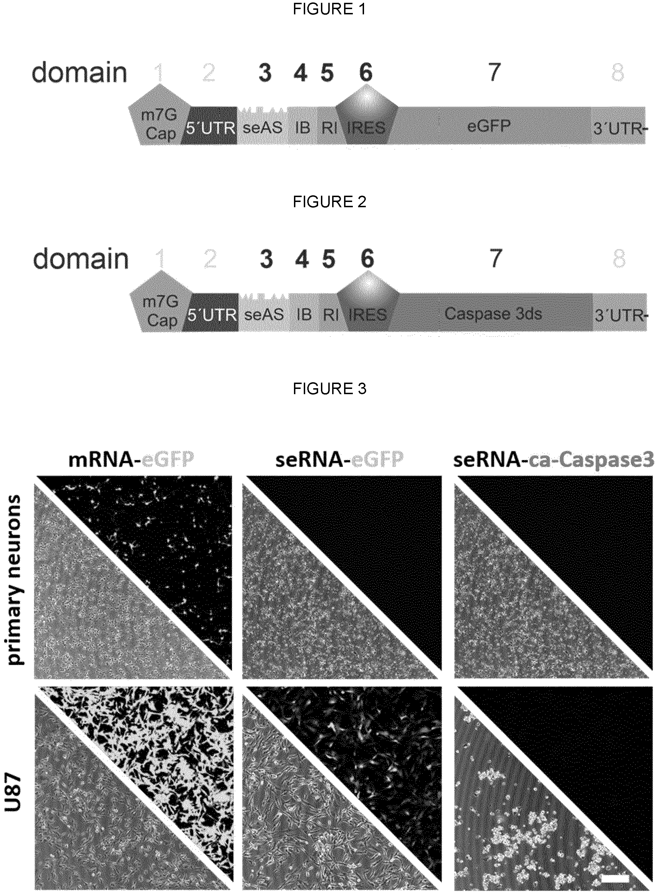

**Figure** 1: shows a schematic representation of an seRNA construct comprising full length seRNA-eGFP multidomain structure in the inactive state. With domain 1 (m7G Cap = 7-methylguanosine cap), Domain 2 and 8 (UTR = untranslated regions) the seRNA comprise classical domains known from mRNAs. Domain 3 (seAS = antisense sequence) consists of an antisense sequence that is complementary to a freely selectable target trigger RNA (here e.g. human keratin). Domain 4 IB = (IRES Blocker) is binding to Domain 6 (IRES = Internal ribosomal entry side) for IRES activity inhibition. Domain 5 (RI = RNase Inhibitor) is located in between. Domain 7 consists of the effector sequence (here e.g. eGFP = enhanced green fluorescent protein).
**Figure 2****:** shows a schematic representation of an seRNA construct comprising full length seRNA-Caspase3ds multidomain structure in the inactive state. With domain 1 (m7G Cap = 7-methylguanosine cap), Domain 2 and 8 (UTR = untranslated regions) the seRNA comprise classical domains known from mRNAs. Domain 3 (seAS = antisense sequence) consists of an antisense sequence that is complementary to a freely selectable target trigger RNA (here e.g. human keratin). Domain 4 IB = (IRES Blocker) is binding to Domain 6 (IRES = Internal ribosomal entry side) for IRES activity inhibition. Domain 5 (RI = RNase Inhibitor) is located in between. Domain 7 consists of the effector sequence (here e.g. Caspase3ds).
**Figure 3****:** shows *in vitro* transcribed seRNAs, transfected into non-target (primary neurons) and U87 glioblastoma cells and analyzed for GFP expression and cell death. As control, a commercial GFP-mRNA was used.

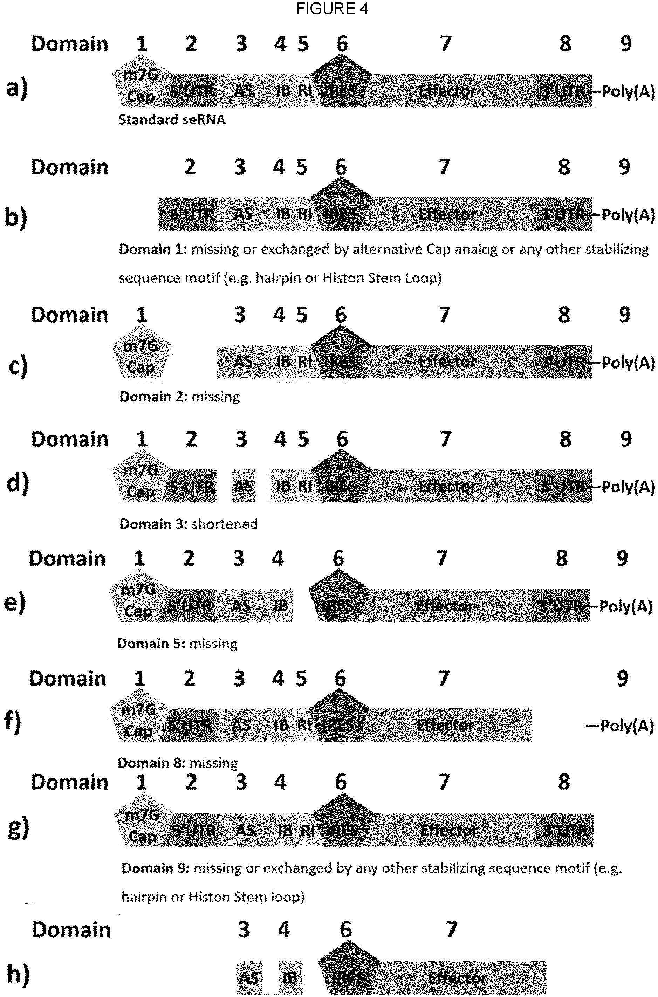

**Figure 4****:** showing possible seRNA constructs in accordance with the invention. seRNA motifs or functional features are denoted from 1 to 9, with domain 1 (Cap Structure as defined herein above), Domain 2 (5 ` UTR = untranslated regions) the seRNA comprise classical domains known from mRNAs. Domain 3 (seAS = antisense sequence) comprises an antisense sequence that is complementary to a freely selectable target trigger RNA. Domain 4 IB = (IRES Blocker) is binding to Domain 6 (IRES = Internal ribosomal entry side) for IRES activity inhibition. Domain 5 (RI = RNase Inhibitor) is located in between. Domain 7 consists of the effector sequence (here coding sequence for a protein to be translated); Domain 8 being a 3' UTR, and domain 9 being poly A tail. All combinations of b) to g) variations are possible, preferred combination of shown embodiments b, d and g and b, d, e and g. Version h) shows an (preferred) embodiment with the shortest seRNA in accordance with the invention.

**SEQ ID NO**: 1 shows a (RNA molecules contain uracil instead of thymine) DNA converted sequence of the seRNA-Caspase3ds construct: 
TGATGTCGGCCTCCACGCTCTGGCGCAGGGCCAGCTCATTCTCATACTTGAGCCTGAAGTC
GTCCGCAGCCAGCCTGGCATTGTCAATCTCCAGGATGACCCGGTTGTTTTCAATGGTGGCG
 
GTCAGGATCTTGTCCCGGAGCTCTTCAATGGTCTTGTAGTAGGGGCTGTAGTCCCGCTCAG
GGCTAGCTGGGCTCTGCTTCAGGTGCCAGTCACGGATCTTCACCTCCAGGTCAGCGTTGG
CCTCCTCCAGGGCGCGCACCTTCTCCAGGTAGGAAGCCAGGCGGTCGTTGAGGTTCTGC
ATGGTGATCTTCTCATTGCCAGTGAGGAGGCCGCCATCACAAGCACCAAAGTCAACAAAGC
CACCAGCAAAACCCCCACCAAAGCCACCTCCAAGGCCACCTCCATAGCCACCTCCAAGGC
CACCTCCATAGCCACCTCCAAAGCCACTACCAGCCCCTCCACCAAAACCACAGCTCACGCC
GCCTCCATAGCCCCCAGCTGATCCCCCAGACACAAACCGAGTTGAACAGGTAGAGACACC
ACGGCCTCCTCCCAGCTGGCAAGAGCCACCCCCGAAACCACCTCCATAGCTGGCAGAGGA
GCTCTGCAGGCGGAGGCTCATGAATTCGTTATGTTATGAACTGCTTCCTTCACGACATTCAA
CAGACCTTGTTATGTTATTAAAAGTCAGGTCGGATCAAGCCATAGTACGGAAAAAACTATGCT
ACCTGTGAGCCCCGTCCAAGGACGTGCGGCCGCTAAAAGAAGTCAGGCCATCACAAATGC
CACAGCTTGAGTAAACTGTGCAGCCTGTAGCTCCACCTGAGAAGGTGTAAGTTATGTTATGG
ATCCCCCCCCTAACGTTACTGGCCGAAGCCGCTTGGAATAAGGCCGGTGTGCGTTTGTCTA
TATGTTATTTTCCACCATATTGCCGTCTTTTGGCAATGTGAGGGCCCGGAAACCTGGCCCTG
TCTTCTTGACGAGCATTCCTAGGGGTCTTTCCCCTCTCGCCAAAGGAATGCAAGGTCTGTT
GAATGTCGTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAAAGCC
ACGTGTATAAGATACACCTGCAAAGGCGGCACAACCCCAGTGCCACGTTGTGAGTTGGATA
GTTGTGGAAAGAGTCAAATGGCTCTCCTCAAGCGTATTCAACAAGGGGCTGAAGGATGCCC
AGAAGGTACCCCATTGTATGGGATCTGATCTGGGGCCTCGGTGCACATGCTTTACATGTGTT
TAGTCGAGGTTAAAAAACGTCTAGGCCCCCCGAACCACGGGGACGTGGTTTTCCTTTGAAA
AACACGATGATAATATGGCCACAACCATGTCTAGAATGATTGAGACAGACAGTGGTGTTGAT
GATGACATGGCGTGTCATAAAATACCAGTGGAGGCCGACTTCTTGTATGCATACTCCACAGC
ACCTGGTTATTATTCTTGGCGAAATTCAAAGGATGGCTCCTGGTTCATCCAGTCGCTTTGTG
CCATGCTGAAACAGTATGCCGACAAGCTTGAATTTATGCACATTCTTACCCGGGTTAACCGA
AAGGTGGCAACAGAATTTGAGTCCTTTTCCTTTGACGCTACTTTTCATGCAAAGAAACAGAT
TCCATGTATTGTTTCCATGCTCACAAAAGAACTCTATTTTTATCACGATGAAGTTGATGGGGG
ATCCCCCATGGAGAACACTGAAAACTCAGTGGATTCAAAATCCATTAAAAATTTGGAACCAA
AGATCATACATGGAAGCGAATCAATGGACTCTGGAATATCCCTGGACAACAGTTATAAAATGG
ATTATCCTGAGATGGGTTTATGTATAATAATTAATAATAAGAATTTTCATAAGAGCACTGGAATG
ACATCTCGGTCTGGTACAGATGTCGATGCAGCAAACCTCAGGGAAACATTCAGAAACTTGA
AATATGAAGTCAGGAATAAAAATGATCTTACACGTGAAGAAATTGTGGAATTGATGCGTGATG
TTTCTAAAGAAGATCACAGCAAAAGGAGCAGTTTTGTTTGTGTGCTTCTGAGCCATGGTGAA
GAAGGAATAATTTTTGGAACAAATGGACCTGTTGACCTGAAAAAAATAACAAACTTTTTCAGA
GGGGATCGTTGTAGAAGTCTAACTGGAAAACCCAAACTTTTCATTATTCAGGCCTGCCGTGG
TACAGAACTGGACTGTGGCATTGAGACAGACTGA
**SEQ ID NO**: 2 shows a DNA converted sequence for the seRNA-eGFP construct: 
TGATGTCGGCCTCCACGCTCTGGCGCAGGGCCAGCTCATTCTCATACTTGAGCCTGAAGTC
GTCCGCAGCCAGCCTGGCATTGTCAATCTCCAGGATGACCCGGTTGTTTTCAATGGTGGCG
GTCAGGATCTTGTCCCGGAGCTCTTCAATGGTCTTGTAGTAGGGGCTGTAGTCCCGCTCAG
GGCTAGCTGGGCTCTGCTTCAGGTGCCAGTCACGGATCTTCACCTCCAGGTCAGCGTTGG
CCTCCTCCAGGGCGCGCACCTTCTCCAGGTAGGAAGCCAGGCGGTCGTTGAGGTTCTGC
ATGGTGATCTTCTCATTGCCAGTGAGGAGGCCGCCATCACAAGCACCAAAGTCAACAAAGC
CACCAGCAAAACCCCCACCAAAGCCACCTCCAAGGCCACCTCCATAGCCACCTCCAAGGC
CACCTCCATAGCCACCTCCAAAGCCACTACCAGCCCCTCCACCAAAACCACAGCTCACGCC
GCCTCCATAGCCCCCAGCTGATCCCCCAGACACAAACCGAGTTGAACAGGTAGAGACACC
ACGGCCTCCTCCCAGCTGGCAAGAGCCACCCCCGAAACCACCTCCATAGCTGGCAGAGGA
GCTCTGCAGGCGGAGGCTCATGAATTCGTTATGTTATGAACTGCTTCCTTCACGACATTCAA
CAGACCTTGTTATGTTATTAAAAGTCAGGTCGGATCAAGCCATAGTACGGAAAAAACTATGCT
ACCTGTGAGCCCCGTCCAAGGACGTGCGGCCGCTAAAAGAAGTCAGGCCATCACAAATGC
CACAGCTTGAGTAAACTGTGCAGCCTGTAGCTCCACCTGAGAAGGTGTAAGTTATGTTATGG
ATCCCCCCCCTAACGTTACTGGCCGAAGCCGCTTGGAATAAGGCCGGTGTGCGTTTGTCTA
TATGTTATTTTCCACCATATTGCCGTCTTTTGGCAATGTGAGGGCCCGGAAACCTGGCCCTG
TCTTCTTGACGAGCATTCCTAGGGGTCTTTCCCCTCTCGCCAAAGGAATGCAAGGTCTGTT
GAATGTCGTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAAAGCC
ACGTGTATAAGATACACCTGCAAAGGCGGCACAACCCCAGTGCCACGTTGTGAGTTGGATA
GTTGTGGAAAGAGTCAAATGGCTCTCCTCAAGCGTATTCAACAAGGGGCTGAAGGATGCCC
AGAAGGTACCCCATTGTATGGGATCTGATCTGGGGCCTCGGTGCACATGCTTTACATGTGTT
TAGTCGAGGTTAAAAAACGTCTAGGCCCCCCGAACCACGGGGACGTGGTTTTCCTTTGAAA
AACACGATGATAATATGGCCACAACCATGTCTAGAATGGTGAGCAAGGGCGAGGAGCTGTT
CACCGGGGTGGTGCCCATCCTGGTCGAGCTGGACGGCGACGTAAACGGCCACAAGTTCA
GCGTGTCCGGCGAGGGCGAGGGCGATGCCACCTACGGCAAGCTGACCCTGAAGTTCATC
TGCACCACCGGCAAGCTGCCCGTGCCCTGGCCCACCCTCGTGACCACCCTGACCTACGG
CGTGCAGTGCTTCAGCCGCTACCCCGACCACATGAAGCAGCACGACTTCTTCAAGTCCGC
CATGCCCGAAGGCTACGTCCAGGAGCGCACCATCTTCTTCAAGGACGACGGCAACTACAA
GACCCGCGCCGAGGTGAAGTTCGAGGGCGACACCCTGGTGAACCGCATCGAGCTGAAGG
GCATCGACTTCAAGGAGGACGGCAACATCCTGGGGCACAAGCTGGAGTACAACTACAACA
GCCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCC
CCATCGGCGACGGCCCCGTGCTGCTGCCCGACAACCACTACCTGAGCACCCAGTCCGCC
CTGAGCAAAGACCCCAACGAGAAGCGCGATCACATGGTCCTGCTGGAGTTCGTGACCGCC
GCCGGGATCACTCTCGGCATGGACGAGCTGTACAAGTAA
**SEQ ID NO**: **3** shows a 5' stabilization motif: 
GGGCCGTCCGGGCAATTGCCCGGACGGCCCCCCGGCAGCCCGCAATTGCGGGCTGCCG
GG (SEQ ID NO: 3;
**SEQ ID NO: 4** shows a 5' stabilization motif:

GCGCGCCAGGGCGCCAATTGGCGCCCTGGCGCGCCCGCGGGTGGGCGCCAATTGGCGC
CCACCCGCGG (SEQ ID NO: 4).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

Based on given data, transfection with seRNA-encoding DNA expression plasmids proved selective functionality of the seRNA platform technology. To enable broad applicability, we additionally tested in vitro transcribed full-length seRNA-ca-caspase and seRNA-GFP RNA molecules in U87 target and non-target primary neurons (Figures 1 and 2). Sequences of the used constructs are provided as SEQ ID NO: 1 and 2. The sequences are shown as DNA sequences and contain as RNA molecules uracil instead of the indicated thymine bases.

As a result, enhanced transfection efficiencies with just one single transfection compared to plasmid transfer were combined with enhanced effector expression intensities in target cells only, to result in bright GFP signals for seRNA-GFP and high targeted cell toxicity upon use of seRNA-ca-caspase RNA. In contrast, no seRNA activation and therefore complete absence of GFP expression or cytotoxicity was detectable in non-target cells, despite efficient transfection efficiencies as shown for control GFP-mRNAs (Figure 3).

In addition, seRNA molecules can be delivered as seRNA encoding plasmid or in vitro transcribed RNA. This results in the broadest possible range of delivery options.

seRNA-GFP and seRNA-ca-caspase coding sequences were cloned into pBluescript SK-vector. Plasmids were linearized and used as templates for T3 primer driven in vitro RNA synthesis using mMESSAGE mMACHINE T3 High Yield Capped RNA Transcription Kit (Thermo Fisher, TSA). In vitro transcribed RNA was purified by Phenol:chloroform extraction and isopropanol precipitation using TRIzol Reagent (Thermo Fisher, TSA). As positive RNA transfection control, stabilized eGFP mRNA was used (CleanCap EGFP mRNA, TriLink Biotechnologies, TSA).

RNA was transfected using Lipofectamine 3000 as described above, just without use of P3000 reagent. For optimal comparison of transferred RNA copies we transfected twice the amount of double-sized seRNA (2 µg) compared to control eGFP mRNA.

## Claims

1. **A method for a targeted expression of a polypeptide of interest in a eukaryotic target cell,** the method comprising the steps of:
(i) Providing an RNA construct, wherein the RNA construct has at least the following sequence elements (a), (b), (c) and (d) in 5' to 3' direction, wherein
(a) is an antisense element comprising a sequence that is complementary to at least a portion of a trigger RNA (endogenously) expressed by the eukaryotic target cell;
(b) is a blocking element that interacts with at least a portion of the translation initiator element (c) at the 3' position;
(c) is a translation initiator element;
(d) is a coding element coding for the polypeptide of interest,
(ii) Directly introducing the RNA construct into a plurality of eukaryotic cells, wherein the plurality of eukaryotic cells comprises at least one eukaryotic target cell and at least one eukaryotic non-target cell;
wherein the blocking element (b) inhibits the function of the translation initiator element (c) in absence of the trigger mRNA in a eukaryotic cell, and is sterically displaced and thereby does not inhibit the function of the translation initiator element (c) in presence of the trigger mRNA in a eukaryotic cell.

2. The method of claim 1, wherein the plurality of eukaryotic cells is **characterized in that** the eukaryotic target cell compared to the eukaryotic non-target cell expresses higher levels of the trigger RNA; preferably wherein a higher levels is an at least 2 fold higher expression of the trigger RNA, preferably at least an expression that is selected from a 3 fold, 4 fold, 5 fold, 6 fold, 7 fold, 8 fold or higher expression.

3. The method of any one of claims 1 to 2, wherein the eukaryotic target cell and eukaryotic non-target cell are two independent cellular entities (preferably two different cell types) or wherein the eukaryotic target cell and eukaryotic non-target cell are the same cellular entity or cell type but at different time points and/or under different conditions.

4. The method of any one of claims 1 to 3, wherein the construct does not comprise a nuclease resistant or inhibitory element, preferably wherein the construct does not comprise a hairpin between elements (b) and (c).

5. The method of any one of claims 1 to 4, which does not comprise a 5' end modification, preferably does not comprise 5' cap.

6. The method of any one of claims 1 to 5, wherein the RNA construct comprises in its sequence at least one modified nucleotide.

7. The method of any one of claims 1 to 6, wherein translation initiator element is a synthetic or wild type IRES sequence.

8. The method of claim 7, wherein the synthetic or wild-type IRES sequence is selected from the group consisting of encephalomyocarditis virus (ECMV) IRES, Coxackievirus B3 (CVB3) IRES, Taura Syndrome Virus (TSV) IRES, Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg marburgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus), eukaryotic IRES derived from Homo sapiens, Aplysia californica, Canis lupus familiaris, Drosophila melanogaster, Gallus gallus, Mus pahari, Mus musculus, Saccharomyces cerevisiae, Zea mays, Rattus norvegicus, Ovis aries, SYTE-IRES1, SYTE-IRES2, and combinations thereof.

9. The method of any one of claims 1 to 8, wherein the RNA construct does not comprise an upstream open reading frame (uORF).

10. The method of any one of claims 1 to 9, wherein the antisense element is capable of specifically binding to the trigger RNA.

11. The method of claim 10, wherein the antisense element does not bind to a poly A tail of the trigger RNA.

12. **A nucleic acid template vector** suitable for in-vitro expressing an RNA construct recited in any one of claims 1 to 11.

13. The nucleic acid template vector of claim 12, which is a DNA or RNA vector.

14. **A pharmaceutical or vaccine composition**, comprising the RNA construct recited in any one of claims 1 to 13, together with a suitable carrier and/or excipient.

15. **An RNA construct for use in the treatment or prevention of a disease** in a subject, wherein the RNA construct is as recited in any one of claims 1 to 11, and wherein the protein of in interest is an antigen suitable for vaccination against the disease to be prevented or is a protein involved with the pathology of a disease to be treated.
